# EUROPEAN PATENT APPLICATION

(11) **EP 4 393 438 A2**
(43) Date of publication of application: **03.07.2024**
(21) Application number: 23220743.1
(22) Date of filing: 29.12.2023
(51) Int. Cl.: A61B 18/14, A61B 34/20

(54) **POSITION AND FORCE SENSORS FOR CATHETERS**

(30) Priority: 30.12.2022 US 202263477944 P; 09.11.2023 US 202318388303
(71) Applicant: BIOSENSE WEBSTER (ISRAEL) LTD., 2066717 Yokneam (IL)
(72) Inventor: ROSEBERRY, Jacob, Irvine, 92618 (US); SUAREZ, Paul, Irvine, 92618 (US); AGNEW V, William James, Irvine, 92618 (US); VAN NIEKERK, Pieter Emmelius, Irvine, 92618 (US); NGUYEN, Thanh, Irvine, 92618 (US); PATEL, Amar, Irvine, 92618 (US); THALER, Sean D., Irvine, 92618 (US); RODRIGUEZ, Jasson, Irvine, 92618 (US)
(74) Representative: Carpmaels & Ransford LLP

(57) **Abstract**

The disclosed technology includes a medical end effector comprising one or more spines extending along a longitudinal axis. The spines further comprising a first surface being outward from the longitudinal axis; one or more contact pads affixed to a portion of the first surface of the spine; and one or more flexible circuits disposed over respective ones of the one or more contact pads, the one or more flexible circuits each comprising an electrode and an inductive coil as one unit.

## Description

### CROSS REFERENCE TO RELATED APPLICATIONS

This application claims the benefit of priority under 35 U.S.C. § 119 to prior filed U.S. Provisional Patent Application No. 63/477,944, filed December 30, 2022, the entire contents of which is hereby incorporated by reference as if set forth in full herein.

### FIELD

The present application relates generally to electronic circuitry, and specifically to electronic circuitry of magnetic field sensors. The present application further relates to catheters including magnetic field sensors.

### BACKGROUND

Cardiac arrhythmias, such as atrial fibrillation (AF), occur when regions of cardiac tissue abnormally conduct electrical signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another.

Many current ablation approaches in the art utilize radiofrequency (RF) electrical energy to heat tissue. RF ablation can have certain risks related to thermal heating which can lead to tissue charring, burning, steam pop, phrenic nerve palsy, pulmonary vein stenosis, and esophageal fistula.

Cryoablation is an alternative approach to RF ablation that generally reduces thermal risks associated with RF ablation. Maneuvering cryoablation devices and selectively applying cryoablation, however, is generally more challenging compared to RF ablation; therefore cryoablation is not viable in certain anatomical geometries which may be reached by electrical ablation devices.

Some ablation approaches use irreversible electroporation (IRE) to ablate cardiac tissue using nonthermal ablation methods. IRE delivers short pulses of high voltage to tissues and generates an unrecoverable permeabilization of cell membranes. Delivery of IRE energy to tissues using multi-electrode catheters was previously proposed in the patent literature. Examples of systems and devices configured for IRE ablation are disclosed in U.S. Patent Pub. No. 2021/0169550A1, 2021/0169567A1, 2021/0169568A1, 2021/0161592A1, 2021/0196372A1, 2021/0177503A1, and 2021/0186604A1, each of which are incorporated herein by reference and attached in the Appendix included in priority application U.S. 63/477,944.

Catheters are commonly used for mapping or ablating cardiac tissue. As will be appreciated, knowing positional information of the catheter can help a physician more accurate perform procedures. A magnetic field can be sensed by positioning a conductive coil in the magnetic field and observing electrical current and/or voltage induced in the coil by a change in the magnetic field that is aligned with an axis of the conductive coil. Because electrical current is induced in the conductive coil, such a coil is also referred to as an inductive coil. Relative position of a sensor including one or more inductive coils can be determined in relation to a known magnetic field source by monitoring the induced electric current and/or voltage.

Navigation sensors which include three coil arrangements aligned along three orthogonal axes to determine position and orientation of the navigation sensor in three dimensions within a known induced magnetic field are disclosed for instance in U.S. Patent Numbers 10,330,742 and 10,677,857, each included by reference as if set forth herein and attached in the Appendix included in priority application U.S. 63/477,944.

Integrating a navigation sensor into a catheter suitable for intravascular and/or intracardiac treatments can involve crafting the sensor by hand, which can result in significant manufacturing time and labor costs. Navigation sensor components can account for a significant percentage of catheter material costs.

Further, as will be appreciated, knowing where and in what direction the force is applied to the catheter can help a physician more accurately place the catheter and ensure sufficient contact is made between the electrodes on the catheter and the tissue. What is needed, therefore, are systems and methods for simplifying circuitry for mapping and ablating cardiac tissue including circuitry for determining the magnitude and direction of a force applied to various points of the catheter.

### SUMMARY

There is provided, in accordance with an example of the disclosed technology, a medical end effector. The medical effector can include one or more spines extending along a longitudinal axis. The spines can include a first surface being outward from the longitudinal axis. The spines can include one or more contact pads affixed to a portion of the first surface of the spine. The spines can include one or more flexible circuits disposed over respective ones of the one or more contact pads. The one or more flexible circuits each can include an electrode and an inductive coil as one unit.

The disclosed technology can include a medical end effector. The medical effector can include an expandable basket assembly. The expandable basket assembly can include a plurality of spines extending along a longitudinal axis. The plurality of spines can be configured to bow radially outward from the longitudinal axis when the expandable basket assembly is transitioned from a collapsed form to an expanded form. The expandable basket assembly can include a first single-axis sensor. The expandable basket assembly can include a second single-axis sensor. The expandable basket assembly can include a third single-axis sensor. The first single-axis sensor, second single-axis sensor, and third single-axis sensor can each be coupled to a respective spine of the plurality of spines and can be collectively configured to function as a three-axis sensor. The medical effector can include a processor. The processor can be configured to receive position data from the first single-axis sensor, second single-axis sensor, and third single-axis sensor. The processor can be configured to receive known information pertaining to the physical properties of the spines of the expandable basket assembly. The processor can be configured to calculate a position of the expandable basket assembly and a force applied to the expandable basket assembly based at least in part on the received position data and known information pertaining to the physical properties of the spines.

### BRIEF DESCRIPTION OF THE DRAWINGS

FIG. 1 is a schematic pictorial illustration of a medical system including a medical probe whose distal end includes a basket assembly with electrodes, in accordance with the disclosed technology;
FIG. 2A is a schematic pictorial illustration showing a section view of a spine, in accordance with the disclosed technology;
FIG. 2B is a schematic pictorial illustration showing a plan view of a flexible circuit, in accordance with the disclosed technology;
FIG. 3 is a schematic pictorial illustration of a showing a perspective view of a spine, in accordance with the disclosed technology;
FIG. 4 is a schematic pictorial illustration showing a perspective view of a basket catheter, in accordance with the disclosed technology;
FIG. 5 is a schematic pictorial illustration showing a perspective view of a catheter, in accordance with the disclosed technology;
FIGs. 6A and 6B are schematic pictorial illustrations showing perspective and detail views of a basket catheter, in accordance with the disclosed technology;
FIGs. 7 is a schematic pictorial illustration showing a perspective view of a basket catheter, in accordance with the disclosed technology;
FIGs. 8 is a schematic pictorial illustration showing a perspective view of a basket catheter, in accordance with the disclosed technology;
FIGs. 9A and 9B are schematic pictorial illustrations showing perspective and side views of a basket catheter, in accordance with the disclosed technology;
FIG. 10 is a schematic pictorial illustration showing a side view of a spine, in accordance with the disclosed technology.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%. In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator or physician whereas "distal" indicates a location further away to the operator or physician.

As discussed herein, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "operator" can include a physician, doctor, surgeon, technician, scientist, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode having a high current density and high electric flux density is positioned at a treatment site, and a second electrode having comparatively lower current density and lower electric flux density is positioned remotely from the treatment site.

As discussed herein, the terms "biphasic pulse" and "monophasic pulse" refer to respective electrical signals. "Biphasic pulse" refers to an electrical signal having a positive-voltage phase pulse (referred to herein as "positive phase") and a negative-voltage phase pulse (referred to herein as "negative phase"). "Monophasic pulse" refers to an electrical signal having only a positive or only a negative phase. Preferably, a system providing the biphasic pulse is configured to prevent application of a direct current voltage (DC) to a patient. For instance, the average voltage of the biphasic pulse can be zero volts with respect to ground or other common reference voltage. Additionally, or alternatively, the system can include a capacitor or other protective component. Where voltage amplitude of the biphasic and/or monophasic pulse is described herein, it is understood that the expressed voltage amplitude is an absolute value of the approximate peak amplitude of each of the positive-voltage phase and/or the negative-voltage phase. Each phase of the biphasic and monophasic pulse preferably has a square shape having an essentially constant voltage amplitude during a majority of the phase duration. Phases of the biphasic pulse are separated in time by an interphase delay. The interphase delay duration is preferably less than or approximately equal to the duration of a phase of the biphasic pulse. The interphase delay duration is more preferably about 25% of the duration of the phase of the biphasic pulse.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

The term "temperature rating", as used herein, is defined as the maximum continuous temperature that a component can withstand during its lifetime without causing thermal damage, such as melting or thermal degradation (e.g., charring and crumbling) of the component.

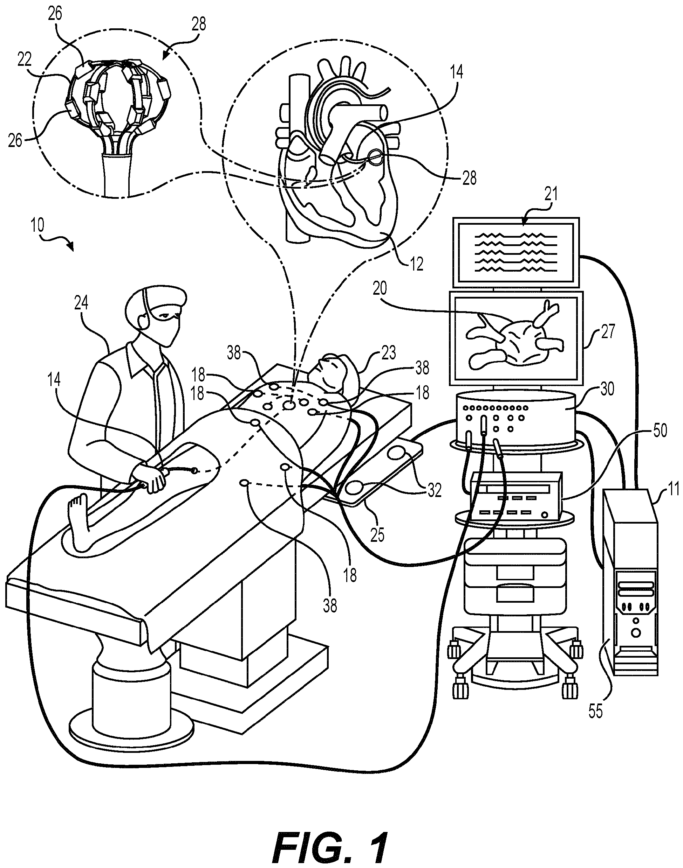

Reference is made to FIG. 1 showing an example catheter-based electrophysiology mapping and ablation system 10. System 10 includes multiple catheters, which are percutaneously inserted by operator 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter 14 that is configured for sensing IEGM is illustrated herein. Operator 24 brings a basket catheter 28 into contact with the heart wall for sensing a target site in heart 12. For ablation, operator 24 would similarly bring a distal end of an ablation catheter to a target site for ablating.

Catheter 14 is an exemplary catheter that includes one and preferably multiple electrodes 26 optionally distributed over a plurality of spines 22 forming a basket assembly 28 at a distal end and configured to sense the IEGM signals. Catheter 14 may additionally include a position sensor 29 embedded in or near the distal tip for tracking position and orientation of basket assembly 28. Optionally and preferably, position sensor 29 is a magnetic based position sensor including three magnetic coils for sensing three-dimensional (3D) position and orientation.

Magnetic based position sensor 29 may be operated together with a location pad 25 including a plurality of magnetic coils 32 configured to generate magnetic fields in a predefined working volume. Real time position of basket assembly 28 of catheter 14 may be tracked based on magnetic fields generated with location pad 25 and sensed by magnetic based position sensor 29. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, each of which are incorporated herein by reference and attached in the Appendix included in priority application U.S. 63/477,944.

System 10 includes one or more electrode patches 38 positioned for skin contact on patient 23 to establish location reference for location pad 25 as well as impedance-based tracking of electrodes 26. For impedance-based tracking, electrical current is directed toward electrodes 26 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182, each of which are incorporated herein by reference and attached in the Appendix included in priority application U.S. 63/477,944.

A recorder 11 displays electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof. The signals may be biphasic or monophasic.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (5) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618 USA.

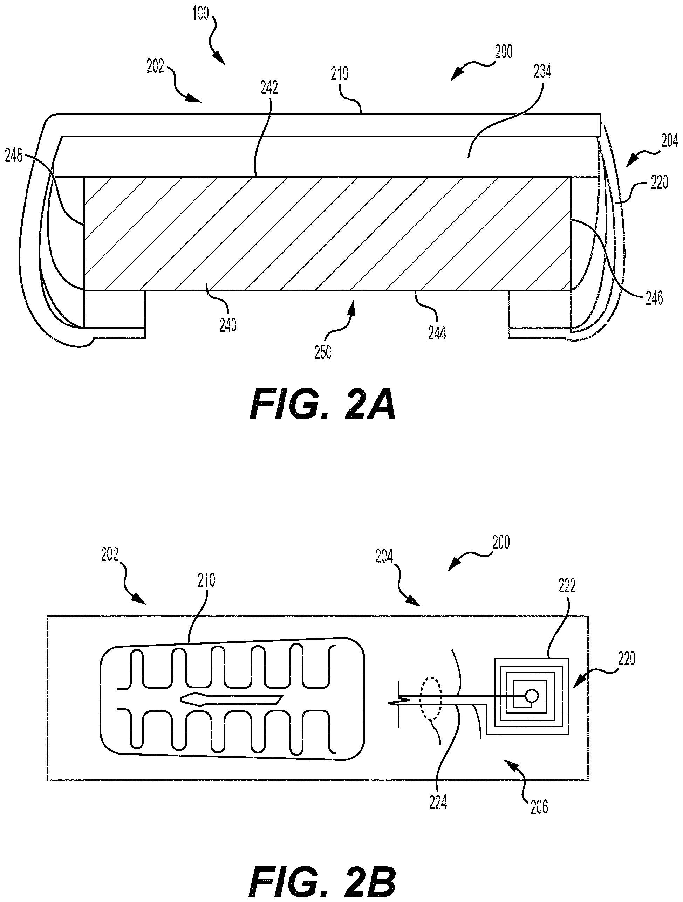

FIG. 2A shows a section view of a spine 100 including a flexible circuit 200. The spine 100 can include a strut 240. The strut 240 can have a generally rectangular cross-section. Alternatively, or in addition, the strut 240 can have a generally circular, oval, elliptical, or polygonal cross-section. The strut 240 can include a first surface 242. A contact pad 230 can be affixed to the first surface 242. The flexible circuit 200 can be disposed over the contact pad 230. For example, as illustrated in FIG. 2A, the flexible circuit 200 can be wrapped partially around the strut 240 and over the contact pad 230. Alternatively, the flexible circuit 200 can be wrapped around the entire strut 240 and over the contact pad 230. In certain embodiments, the contact pad 230 is an insulator, electrically separating a conductive strut 240 from the flexible circuit 200. In other examples the contact pad 230 has one or more surfaces to increase or facilitate the attachment between the strut 240 and/or the flexible circuit 200.

FIG. 2B illustrates a plan view of a flexible circuit 200. The flexible circuit 200 can include both an electrode 210 and an inductive coil 220. For example, the electrode 210 can be an ablation electrode and the inductive coil 220 can be configured to sense magnetic fields, such as magnetic fields generated by magnetic field generator pads located under a patient.

The flexible circuit 200 can be attached to an outer surface of a spine 100, for example using a suitable epoxy or other adhesive. The contact pad 230 can include a long side along the first surface 242 and a short side perpendicular to the long side and the inductive coil 220, when attached to the spine 100, can be disposed solely on at least one of the long side and the short side. The flexible circuit 200 can include a first side 202 and a second side 204. The electrode 210 can be generally disposed on the first side 202 and the inductive coil can be generally disposed on the second side 204. When attached to the spine 100, at least a portion of the first side 202 of the flexible circuit 200 can be generally parallel to the first surface 242. Alternatively, or in addition, at least a portion of the second side 204 can be generally perpendicular to the first surface 242. The strut 240 can further include a second surface 244, a third surface 246, and a fourth surface 248. As illustrated in FIG. 2A, the flexible circuit 200 can be wrapped around the spine over the first surface 242, third surface 246, fourth surface 248, and a portion of the second surface 244. A gap 250 can be formed along the second surface 244. As explained further herein, the gap 250 can be configured to allow one or more wires to be routed, proximate the second surface along the length of the spine 100.

The flexible circuit 200 can be formed with a substrate 206 comprising a suitable dielectric material, such as a polyimide, on which electrical traces can be deposited and etched using printed circuit fabrication techniques that are known in the art.

Prior to attachment of flexible circuit 200 to the outer surface of a spine 100, electrode 210 is formed on the outer side of substrate 206 by depositing and etching a suitable conductive material, such as gold. Electrode 210 can contact tissue in heart 12, such as the tissue of ostium 51. Spiral conductive traces 222 are deposited on substrates 206 in a similar fashion to electrode 210, and serve as inductive coil 220. For example, the spiral conductive traces 222 can be deposited to form a single-axis sensor (SAS). The dimensions of inductive coil 220 are limited by the available space on substrates 206, for example to about 2×2 mm. For enhanced sensitivity, traces 222 typically have a fine pitch, for example 0.4 mm or less, and may be covered by an insulating coating to prevent short-circuiting of the traces by body tissue and fluids. Electrical wiring 224 couples inductive coil 220 to PIU 30, and electrode 210 is coupled by wiring to the PIU 30 in similar fashion. Conductive traces may be formed on both sides of substrate 206, or deposited in multiple layers on the substrate 206, using printed circuit fabrication techniques that are known in the art, to enable connection of wiring 224. The wiring 224 can be routed along the length of the spine 100 in gap 250.

The inductive coils 220 may be operated together with a location pad 25 including a plurality of magnetic coils 32 configured to generate magnetic fields in a predefined working volume. Further the electrode 210 can be configured to be location reference electrodes, such as electrode 26 for impedance-based tracking. As explained above, PIU 30 receives and processes the signals and includes processing capability for implementing real-time computations of location of the catheters and for performing ECG calculations. For example, the PIU 30 can receive and process signals from inductive coils 220 and/or electrodes 210. Alternatively, or in addition, electrode 210 can be an ablation electrode configured to connect to an ablation energy generator 50.

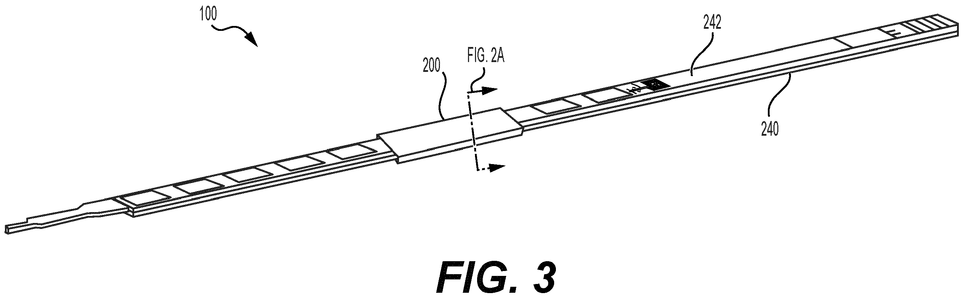

FIG. 3 is a perspective view of a spine 100. As illustrated in FIG. 3, the flexible circuit 200 can wrap around the spine 100 at a location along the length of the spine. Alternatively, a plurality of flexible circuits 200 can be wrapped around a spine 100. Further, the spine 100 can include a plurality of contact pads 230.

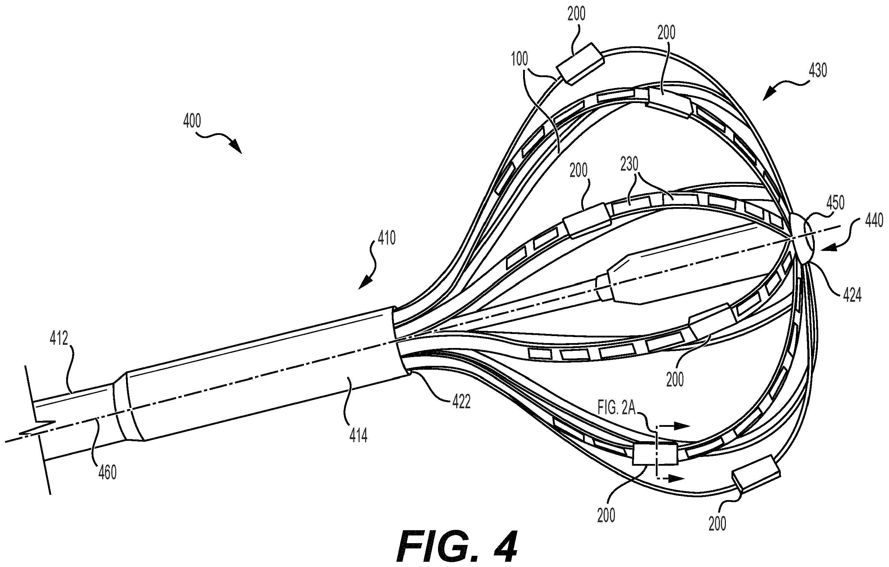

FIG. 4 is a perspective view of a basket catheter assembly 400. Basket assembly 400 can include a plurality of spines 100. For example, the spines 100 can be flexible strips, each of which are coupled the flexible circuit 200. The basket assembly 400 may include any suitable number of flexible circuits 200. In some embodiments, the basket assembly 400 can include ten spines 100 with a flexible circuit 200 attached to each individual spine 100.

The basket assembly 400 can include an insertion tube 410 with a proximal end 412 and a distal end 414. The basket assembly can further include an expandable basket assembly 430 disposed at the distal end 414 of the insertion tube 410. For example, the plurality of spines 100 can be configured to form an expandable basket assembly 430 by extending along a longitudinal axis 460 and attaching on a proximal end to the distal end 414 of the insertion tube 410 and on a distal end to each other or a tip. As illustrated in FIG. 4, the spines 100 can be configured to bow radially outward from the longitudinal axis when the expandable basket assembly 430 is transitioned from a collapsed form to an expanded form.

The basket assembly 400 can further include a three-axis sensor 440. For example, the three axis sensor can be disposed proximate the distal end of the spines 100. Further the basket assembly 400 can include an atraumatic tip 450 disposed at the distal end of the spines 100. The three-axis sense 440 can be configured to function as the atraumatic tip 450.

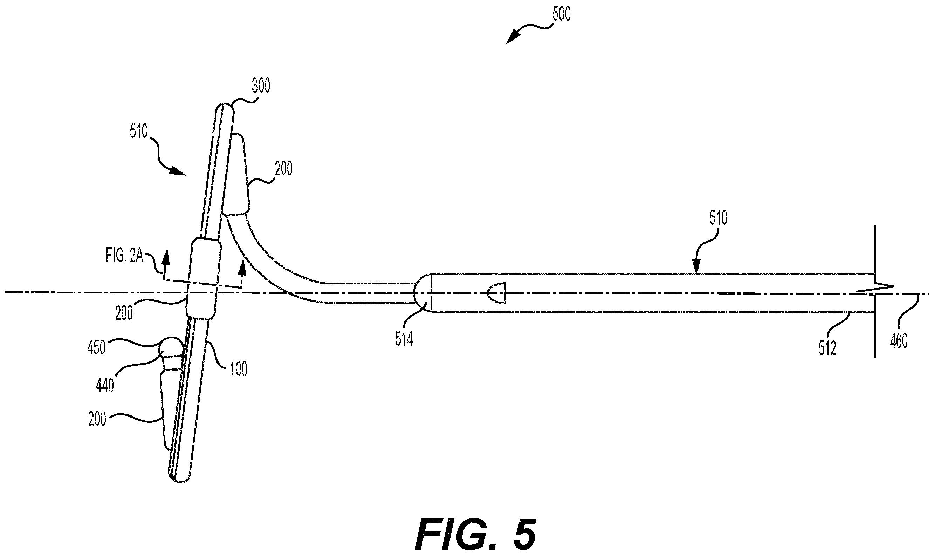

FIG. 5 is a perspective view of a catheter 500. The catheter 500 can include a spine 100. The catheter 500 can include a proximal shaft 520 having a proximal end 512 and a distal end 514. The spine 100 can be disposed at the distal end 414 of the proximal shaft 520. For example, the spine 100 can be coupled to a proximal shaft 520 at the distal end 414. The spine 100 can include a distal portion 510. The spine 100 can have a tubular body. The spine 100 can include one or more flexible circuits 200 which can wrap partially or entirely around the spine 100. For example, as illustrated in FIG. 5, the distal portion 510 of the spine 100 can include three flexible circuits 200 which conform to the curved surface of a tubular spine 100. For example, the inductive coils 220 of the flexible circuits 200 can collectively function as a three axis sensor (TAS) during an intravascular treatment. As illustrated in FIG. 5, the distal portion 510 of the spine 100 can be moved into a generally circular shape when within vasculature or a heart. The generally circular shape can be slightly helical ("lasso"). The circular shape can be generally orthogonal to the longitudinal axis 460 defined by the proximal shaft 520. Alternatively, the circular shape can be aligned to the longitudinal axis 460 or at an oblique angle to the longitudinal axis 460. The circular shape can curve in the clockwise or counterclockwise direction. The flexible circuits can be positioned around the circular shape such that a position and orientation of the distal portion 510 can be determined in three dimensions when the distal portion 510 is within a known varying magnetic field.

The spine 100 of catheter 500 can be further configured to have a delivery configuration in which the distal portion 510 and proximal shaft 520 are aligned along a longitudinal axis 460.

The proximal shaft 520 can have an elongated tubular construction. The proximal shaft 520 can have a single, axial, or central lumen. The proximal shaft 520 can be flexible, i.e., bendable, but substantially non-compressible along its length. The proximal shaft 520 can be of any suitable construction and made of any suitable material. In some examples, the proximal shaft 520 has an outer polymer wall having an interior braided metal mesh. The proximal shaft 520 can have sufficient structural integrity such that when the control handle 101 is rotated, the tubular body 103, including the proximal shaft 106 and distal portion 108, rotate in a corresponding manner. The outer diameter of the proximal shaft 520 is preferably about 8 French or about 7 French.

The useful length of the catheter 500, i.e., that portion that can be inserted into the body, can vary as appropriate based on treatment procedure and anatomy of a patient. For most treatments, the useful length can be about 110 centimeters (cm) to about 120 cm. The length of the distal portion 108 is a relatively small portion of the useful length and preferably is about 3.5 cm to about 10 cm, and more preferably about 5 cm to about 6.5 cm.

In some examples, the distal portion 510 can have a section aligned with the longitudinal axis 460, when the distal portion 510 is in the substantially circular shape, measuring about 3 millimeters (mm) to about 12 mm. Further, in some examples, the circumference of the circular shape can be modified in patient via manipulation of a control handle. The circular shape can have a circumference measuring about 3 cm to about 8 cm, more preferably about 4 cm to about 6 cm, and more preferably about 5 cm.

The proximal shaft 520 and spine 100 can be joined with glue or the like. In some examples, the junction can include a spacer similar to as describe in U.S. Patent No. 5,964,757 incorporated herein by reference in its entirety and attached in the Appendix included in priority application U.S. 63/477,944.

The catheter 500 can further include a three-axis sensor 440. For example, the three axis sensor 440 can be disposed proximate the distal end of the spines 100. Further the basket assembly 400 can include an atraumatic tip 450 disposed at the distal end of the spines 100. The three-axis sensor 440 can be configured to function as the atraumatic tip 450.

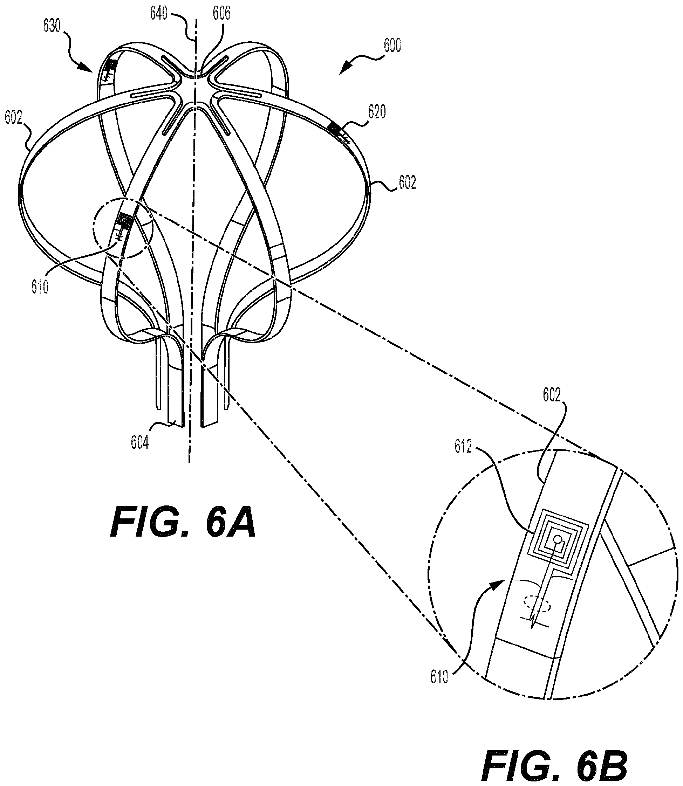

FIGs. 6A and 6B show perspective and detail views of a basket assembly 600. The basket assembly 600 can include a plurality of single axis sensors 610 attached to the spines 602. In particular FIG. 6A illustrates a perspective view and FIG. 6B illustrates a detail view of the basket assembly 600 with a plurality of single axis sensors attached thereto. Although FIGs. 6A and 6B illustrate the basket assembly 600 having the single axis sensors attached to an outwardly-facing surface of the spine 602, one of skill in the art will appreciate that the single-axis sensors can alternatively or additionally be attached to an inwardly-facing surface. All of the features described in relation to the basket catheter assembly 400 can be incorporated into the basket catheter 600.

The basket assembly 600 can include a plurality of single axis sensors (SAS). For example, as illustrated in FIG. 6A, the basket assembly can include a first single-axis sensor 610, a second single-axis sensor 620, a third single-axis sensor 630. The single axis-sensors can be inductive coils to sense magnetic fields generated by magnetic field generators, such as the magnetic coils 32 discussed herein. For example, as illustrated in the detail view of the first single-axis sensor 610, it can include an inductive coil 612.

The first single-axis sensor 610, the second single axis sensor 620, and the third single axis sensor 630 can be collectively configured to function as a three-axis sensor (TAS). For example, the single-axis sensors can be distributed equidistant around a longitudinal axis 640. As illustrated in FIG. 6, for a basket assembly 600 with 6 spines, every other spine can include a SAS, for a total of three SASs that can function as a TAS. For example, the first single-axis sensor 610, the second single axis sensor 620, and the third single axis sensor 630 can be distributed equidistant around a longitudinal axis 640. Further, the first single-axis sensor 610, the second single axis sensor 620, and the third single axis sensor 630 can be located generally at the equator of the basket assembly 600. Alternatively, the single-axis sensors can be distributed nearer to the distal end 606 or nearer to the proximal end 604 of the basket assembly 600.

By functioning as a three-axis sensor, the three single-axis sensors can be used to accurately calculate the position of the expandable basket assembly 600 and the force applied to the expandable basket assembly 600. For example, the position data from the single-axis sensors forming a three-axis sensor and known information pertaining to the physical properties of the basket assembly 600 can be utilized to calculate force on the basket assembly 600 based on change in position caused by deformation of the basket assembly 600.

The workstation 55 can be configured to know certain physical properties of the basket assembly 600, such as the spring constant K of the spines 602. The workstation 55 can further be configured to receive position information from the single-axis sensors forming a three-axis sensor and calculate both location and deflection of the basket assembly 600. In this way, the workstation 55 can calculate both location of the basket assembly as well as force applied to the basket assembly (i.e., by calculating force using the deflection information and the known physical properties of the basket assembly 600). Further, the workstation 55 can be configured to calculate the deflection of one or more of the individual spines 602 of the basket assembly 600 based at least in part on the location information of the basket assembly 600 calculated from the received position data from the three SAS functioning as a TAS. In this way, the workstation can calculate the force applied to an individual spine 602 based on at least the calculated deflection and the known information pertaining to the physical properties of the spine such as the spring constant K of the spine 602 (e.g., by using Hooke's law, or the equation F=d*K).

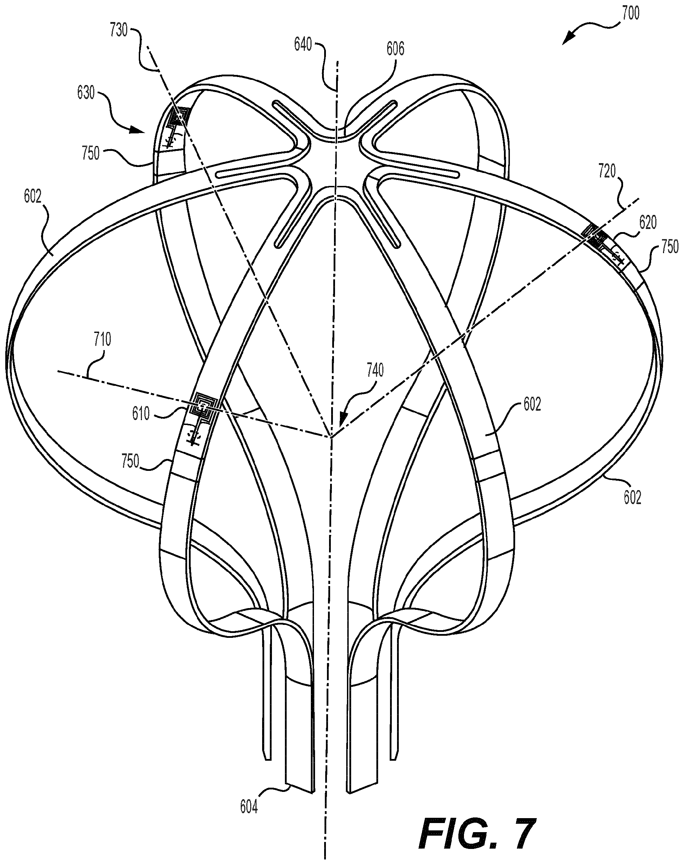

FIG. 7 is a perspective view of another example of a basket assembly 700. All of the features described in relation to the basket catheter assemblies 400 and 600 can be incorporated into the basket catheter 700. As illustrated in FIG. 7, the three SAS sensors can form a TAS by the inductive coils of the SAS sensors spiraling around a coil axis that all converge at a referential datum 740. For example, the first SAS 610 can include a first coil axis 710 around which the inductive coil of the first SAS spirals. Similarly, the second SAS 620 and third SAS 630 can also include a respective second coil axis 720 and third coil axis 730 around which the respective inductive coils of the second SAS 620 and third SAS 630 spiral around. The referential datum 740 where the coil axes converge can be a known location within the catheter basket assembly 700. Further, the referential datum 740 location can be used to calculate the position of and force on the basket assembly 700.

As illustrated in FIG. 7, the basket assembly 700 can further include one or more force sensors 750. For example, the force sensors can be strain gauges. By including a plurality of force sensors 750 attached to the spines 602, the disclosed technology can be configured to more accurately detect a force applied to the basket assembly 700 than basket assemblies 600 having only the three SAS functioning as a TAS as illustrated in FIG. 6. For example, the workstation 55 can be configured to know which force sensor 750 is attached to which spine 22 (i.e., the workstation 55 can be programmed to correlate a signal received from a given force sensor 750 to an assigned spine 602 of the basket assembly 700). In this way, the workstation 55 can receive force signals from each of the force sensors 750 and determine at least the direction of the force at each spine 602 based on signals received from the assigned force sensor 750 attached to a given spine 602. In other words, the workstation 55 can be configured to detect at least a direction of force applied to each spine 602 individually.

For example, as a force is applied to a first side of the basket assembly 700, the degree to which each force sensor 750 is either compressed or strained can indicate where the force is originating from. To illustrate, the spine 602 nearest the location where the force is applied is more likely to compress on an outwardly-facing surface while the outwardly-facing surface of a spine 602 located further away from the location of the force is more likely to stretch as the force is applied. Thus, by correlating the type of force (compressive or tensile) with the known position of the spine 602 on the basket assembly 700, the force sensors 750 can be configured to detect at least the direction of a force applied to the basket assembly 700.

The contact force sensors 750 can be sized and position as would be suitable for the particular application. For example, although the plurality of force sensors 750 are shown as having a given length and positioned on the spine 602 at the given illustrated positions, one of skill in the art will appreciate that the force sensors 750 can be larger or smaller than those shown in the figures. Furthermore, although FIG. 7 illustrate only a single force sensor 750 attached to a single spine 602, the disclosed technology can include multiple force sensors 750 attached to a given spine 602. Furthermore, the force sensors 750 can be attached to the spine 602 using any suitable method. For example, the force sensors 750 can be attached to the spine 602 using adhesive, fasteners, crimps, etc.

Further, combining the directional information determined from the force sensors 750 with the position and force information calculated from the three SAS functioning as a TAS and the known information pertaining to the physical properties of the basket assembly 700, the workstation 55 can be configured to determine how much force is applied to each spine 602, and determine a direction of the force applied to the basket assembly 700.

Thus, by correlating the direction of the force detected by a given force sensor 750 with the calculated position and force applied to the basket assembly 700, the disclosed technology can be configured to detect the magnitude and direction of a force applied to the basket assembly 700. The disclosed technology can further determine a difference in the force applied to a first side of the basket assembly 700 and a second side of the basket assembly 700. Furthermore, the disclosed technology can determine the magnitude and direction of the force applied to the individual spines 602 of the basket assembly 700. As will be appreciated, knowing the magnitude and direction of force on the basket assembly 700 can be helpful to determine whether the electrodes (such as electrodes 820, discussed further herein) have made sufficient contact with tissue.

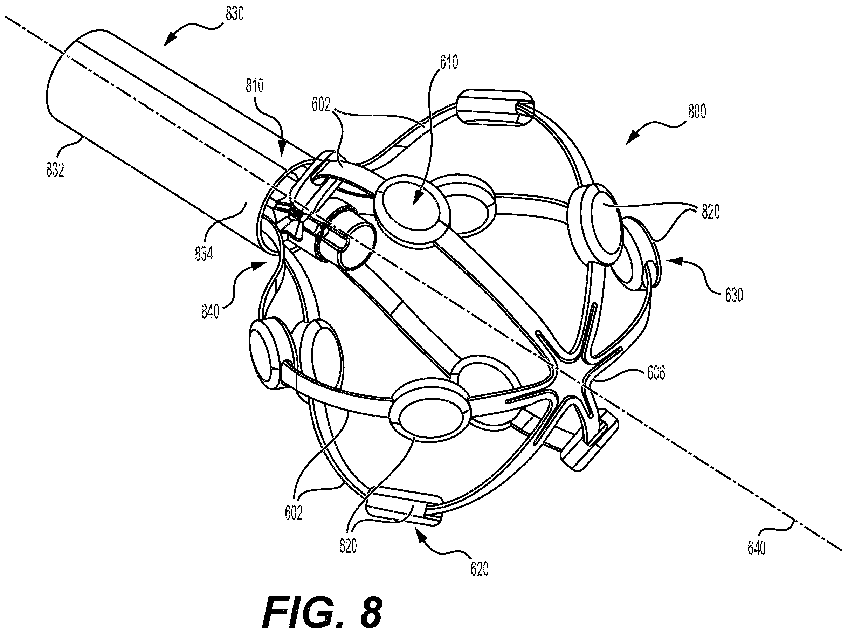

FIG. 8 is a schematic pictorial illustration showing a perspective view of a medical end effector having a basket assembly 800 in an expanded form when unconstrained, such as by being advanced out of an insertion tube 830 at a distal end 834. The insertion tube having a proximal end 832 and a distal end 834. In the expanded form (FIG. 8), the spines 602 bow radially outwardly along a longitudinal axis 640. All of the features described in relation to the basket catheter assemblies 400, 600, and 700 can be incorporated into the basket catheter 800.

As shown in FIG. 8, basket assembly 800 includes a plurality of flexible spines 602 that are formed at the end of a tubular shaft 840 and are connected at both ends. During a medical procedure, operator 24 can deploy basket assembly 800 by extending tubular shaft 840 from insertion tube 830 causing the basket assembly 800 to exit the insertion tube 830 at the distal end 834 and transition to the expanded form. Spines 602 may have elliptical (e.g., circular) or rectangular (that may appear to be flat) cross-sections, and include a flexible, resilient material (e.g., a shape-memory alloy such as nickel-titanium, also known as Nitinol) forming a strut as will be described in greater detail herein.

In examples described herein, electrodes 820 can be configured to deliver ablation energy (RF and/or IRE) to tissue in heart 12. In addition to using electrodes 820 to deliver ablation energy, the electrodes can also be used to measure a physiological property such as local surface electrical potentials at respective locations on tissue in heart 12. The electrodes 820 can be biased such that a greater portion of the electrode 820 faces outwardly from the basket assembly 800 such that the electrodes 820 deliver a greater amount of electrical energy outwardly away from the basket assembly 800 (i.e., toward the heart 12 tissue) than inwardly toward the basket assembly 800.

Examples of materials ideally suited for forming electrodes 820 include gold, platinum, and palladium (and their respective alloys). These materials also have high thermal conductivity which allows the minimal heat generated on the tissue (i.e., by the ablation energy delivered to the tissue) to be conducted through the electrodes to the back side of the electrodes (i.e., the portions of the electrodes on the inner sides of the spines), and then to the blood pool in heart 12.

The insertion tube 830 can further include a TAS 810 coupled to the distal end 834. The workstation 55 can be configured to received position data from the TAS 810 and calculate the magnitude and direction of force on the basket assembly 800 based at least in part on the position data received from the TAS 810. For example, the workstation 55 can calculate the calculate the magnitude and direction of force on the basket assembly 800 using the position data received from the TAS 810, the position data received from the three SAS 610, 620, 630 functioning as a TAS, and the known information pertaining to the physical properties of the basket assembly 800.

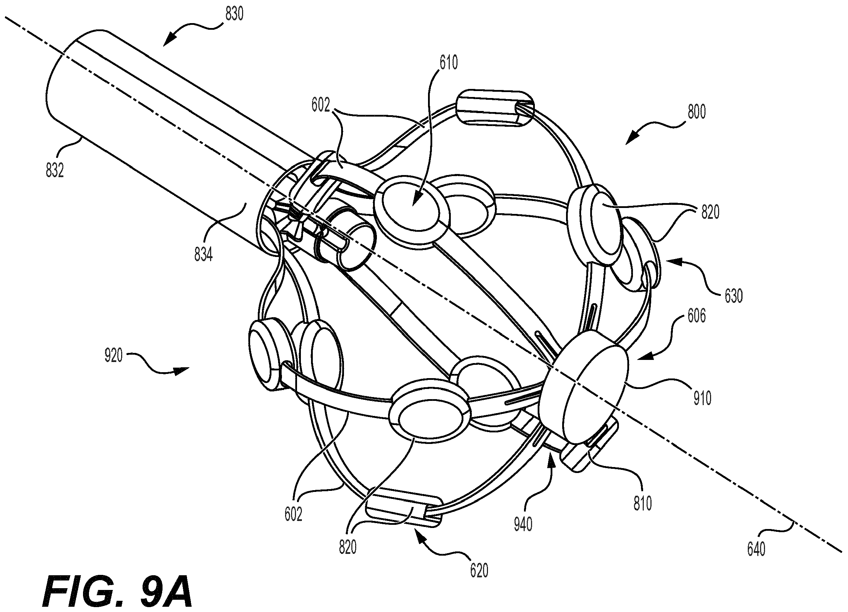

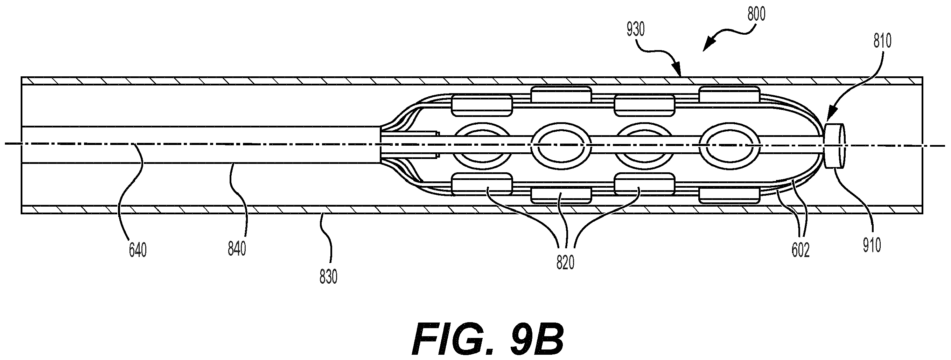

FIGs. 9A and 9B further show perspective and side views of a basket assembly 800 showing the expanded form 920 and collapsed form 930 of basket assembly 800. FIG. 9A is a schematic pictorial illustration showing a perspective view of a medical end effector having a basket assembly 800 in an expanded form when unconstrained, such as by being advanced out of an insertion tube 830 at a distal end 834. FIG. 9B shows the basket assembly in a collapsed form within the insertion tube 830. In the expanded form (FIG. 9A), the spines 602 bow radially outwardly along a longitudinal axis 640 and in the collapsed form (FIG. 9B) the spines are constrained generally along the longitudinal axis 640 of tubular shaft 840.

The basket assembly 800 can include a central intersection 940 at a point where the spines 602 converge near the distal end 606. The basket assembly 800 can include a TAS 810 disposed proximate the central intersection 940. As explained above, the TAS 810 can be used to calculate the position of the basket assembly 800 as well as the magnitude and direction of force on the basket assembly 800. Further the basket assembly 800 can include an atraumatic tip 910 disposed at the distal end 606 of the spines 602 (e.g., at the central intersection 940). The TAS 810 can be configured to function as the atraumatic tip 910.

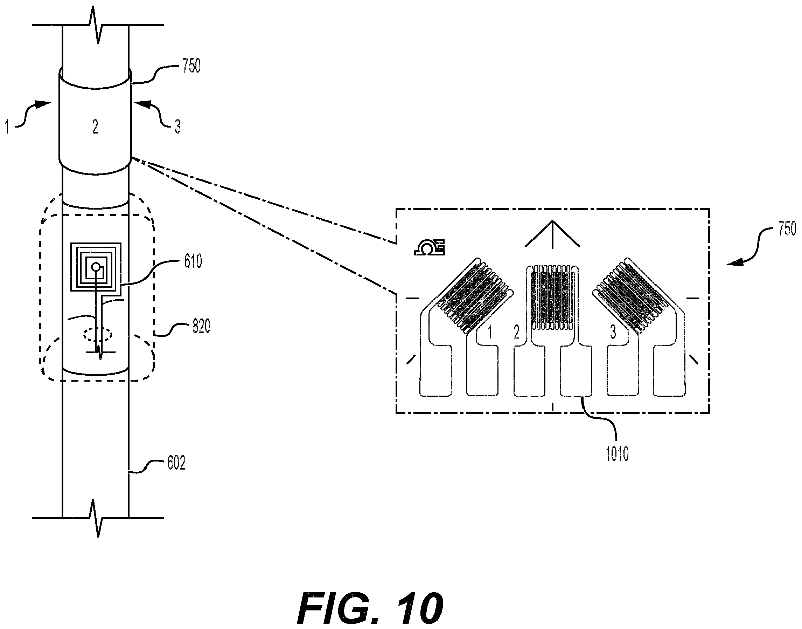

FIG. 10 is a side view of a spine with a force sensor 750. As illustrated in FIG. 10, the force sensor 750 can be a strain gauge 1010. For example, the strain gauge 1010 can be a flat strain gauge having three sensors 1, 2, 3. Alternatively, or in addition, the strain gauge 1010 can include less sensors, such as 1 or 2 sensors, or can include more sensors, such as 4, 5, or 10. The strain gauge 1010 can be a flat strain gauge that is wrapped around the spine 602. For example, as illustrated in FIG. 10, the spine 602 can be tubular in shape and the strain gauge 1010 can wrap around the spine 602 such that the three sensors 1, 2, 3 are spaced generally evenly around the circumference of the spine 602. As will be appreciated, by spacing the three sensors 1, 2, 3 of strain gauge 1010 around the spine 602, the direction of a force being applied to the spine 602 can be determined. Alternatively, or in addition, the force sensor 750 can be a load cell, a piezoelectric sensor, a force sensing resistor, a magnetic force sensor, etc.

Additionally, as illustrated in FIG. 10, the SASs, such as the first SAS 610 can be disposed under the electrodes 820. As will be appreciated, strain on the SASs can be reduced by being installed under the electrodes 820.

Further details of basket catheter technology are described in U.S. Patent No. 7,149,563 and U.S. Pub. Nos. 2018/0344202 and 2021/0059608 each of which are incorporated herein by reference and attached in the Appendix included in priority application U.S. 63/477,944.

The disclosed technology described herein can be further understood according to the following clauses:
Clause 1: A medical end effector, comprising: one or more spines extending along a longitudinal axis, the spines comprising: a first surface being outward from the longitudinal axis; one or more contact pads affixed to a portion of the first surface of the spine; and one or more flexible circuits disposed over respective ones of the one or more contact pads, the one or more flexible circuits each comprising an electrode and an inductive coil as one unit.
Clause 2: The medical end effector according to Clause 1, the inductive coil comprising a first inductive coil embedded in the flexible circuit such that the first inductive coil conforms to the curvature of the flexible circuit bulging over respective ones of the ones of the one or more contact pads.
Clause 3: The medical end effector according to Clause 2, the first inductive coil being confined between two substantially parallel, flexible surfaces.
Clause 4: The medical end effector according to any one of Clauses 1-3, wherein the contact pad comprises a long side along the first surface and a short side perpendicular to the long side and the inductive coil is disposed solely on at least one of the long side and the short side
Clause 5: The medical end effector according to any one of Clauses 1-4, wherein the electrode is disposed on a first side of the flexible circuit and the inductive coil is disposed on a second side of the flexible circuit.
Clause 6: The medical end effector according to Clause 5, wherein at least a portion of the first side of the flexible circuit is disposed generally parallel to the first surface of the spine.
Clause 7: The medical end effector according to Clause 5 or 6, wherein at least a portion of the second side of the flexible circuit is disposed generally perpendicular to the first surface of the spine.
Clause 8: The medical end effector according to any one of Clauses 1-7, wherein each respective one of the one or more flexible circuits extends around the respective spine.
Clause 9: The medical end effector according to any one of Clauses 1-7, wherein the spines further comprise: a second surface opposite the first surface; a third surface disposed generally perpendicular to the first surface; a fourth surface opposite the third surface.
Clause 10: The medical end effector according to Clause 9 further comprising one or more wires disposed proximate the second surface and routed along a length of the spine.
Clause 11: The medical end effector according to Clause 9 or 10, wherein the one or more flexible circuits each extend around the first surface, third surface, fourth surface, and at least a portion of the second surface of the spine.
Clause 12: The medical end effector according to Clause 11, wherein the one or more flexible circuits extending around at least a portion of the second surface of the spine defines a gap in the flexible circuit through which the one or more wires are routed.
Clause 13: The medical end effector according to any one of Clauses 1-11, wherein the inductive coil is configured to function as a single-axis sensor.
Clause 14: The medical end effector according to any one of Clauses 1-13, further comprising: an ablation power generator configured to be connected to the medical probe, and apply an electrical signal to at least one of the electrodes to ablate a tissue of a body part; and a position module configured to receive position signals from at least one of the inductive coils.
Clause 15: The medical end effector of any one of Clauses 1-14, wherein the one or more spines are configured to form an expandable basket assembly comprising the one or more spines extending along the longitudinal axis and configured to bow radially outward from the longitudinal axis when the expandable basket assembly is transitioned from a collapsed form to an expanded form.
Clause 16: The medical end effector of any one of Clauses 1-13 or 15 wherein the one or more spines further comprise: a distal portion; a delivery configuration in which the distal portion is aligned along a longitudinal axis; and a deployed configuration in which the distal portion comprises a generally circular shape generally orthogonal to the longitudinal axis.
Clause 17: The medical end effector of any one of Clauses 1-16 further comprising a three-axis sensor coupled to the one or more spines wherein the one or more spines comprises a distal end and a proximal end and the three-axis sensor is disposed proximate the distal end.
Clause 18: The medical end effector according to Clause 17 further comprises an atraumatic tip disposed distal the three-axis sensor.
Clause 19: A medical end effector comprising: an expandable basket assembly comprising: a plurality of spines extending along a longitudinal axis and configured to bow radially outward from the longitudinal axis when the expandable basket assembly is transitioned from a collapsed form to an expanded form; a first single-axis sensor; a second single-axis sensor; and a third single-axis sensor, the first single-axis sensor, second single-axis sensor, and third single-axis sensor are each coupled to a respective spine of the plurality of spines and are collectively configured to function as a three-axis sensor; and a processor configured to: receive position data from the first single-axis sensor, second single-axis sensor, and third single-axis sensor; receive known information pertaining to the physical properties of the spines of the expandable basket assembly; and calculate a position of the expandable basket assembly and a force applied to the expandable basket assembly based at least in part on the received position data and known information pertaining to the physical properties of the spines.
Clause 20: The medical end effector according to Clause 19, wherein the processor is further configured to: calculate a deflection of the expandable basket assembly at least in part on received position data.
Clause 21: The medical end effector according to Clause 20, wherein the processor is further configured to: calculate a force applied to the expandable basket assembly based at least in part on the calculated deflection of the expandable basket assembly and the known information pertaining to the physical properties of the spines.
Clause 22: The medical end effector according to any one of Clauses 19-21, wherein the processor is further configured to: calculate a deflection of one or more individual spines of the plurality of spines at least in part on received position data.
Clause 23: The medical end effector according to Clause 22, wherein the processor is further configured to: calculate an individual force applied to each of one or more individual spines of the plurality of spines based at least in part on the calculated deflection of one or more individual spines and the known information pertaining to the physical properties of the spines.
Clause 24: The medical end effector according to any one of Clauses 19-23, further comprising a plurality of strain gauges, each strain gauge of the plurality of strain gauges coupled to a spine of the plurality of spines.
Clause 25: The medical end effector according to Clause 24, wherein the processor is further configured to: receive force data from the plurality of strain gauges; and calculate a force and a direction of said force applied to the expandable basket assembly based at least in part on the received force data from the plurality of strain gauges.
Clause 26: The medical end effector according to Clause 24 or 25, wherein each strain gauge of the plurality of strain gauges is configured to wrap around a spine of the plurality of spines and measure strain in three axes.
Clause 27: The medical end effector according to any one of Clauses 19-26, further comprising: an insertion tube extending along the longitudinal axis and having a proximal end and a distal end, wherein the distal end is coupled to the expandable basket assembly; and a three-axis sensor coupled to the distal end of the insertion tube.
Clause 28: The medical end effector of any one of Clauses 19-27, further comprising a three-axis sensor coupled to the expandable basket assembly and positioned distal the distal end of the insertion tube.
Clause 29: The medical end effector of Clause 27 or 28, wherein the processor is further configured to: receive position data from the three-axis sensor; and calculate a force and a direction of said force applied to the expandable basket assembly based at least in part on the receive position data from the three-axis sensor.
Clause 30: The medical end effector according to Clause 28, further comprising an atraumatic tip positioned distal the three-axis sensor.
Clause 31: The medical end effector according to any one of Clauses 19-30, wherein the known information pertaining to the physical properties of the spine comprises a spring constant of each spine.
Clause 32: The medical end effector according to any one of Clauses 19-31, wherein the plurality of spines in the expanded form comprises a generally circular shape generally orthogonal to the longitudinal axis.
Clause 33: The medical end effector according to Clause 32, the first single-axis sensor, second single-axis sensor, and third single-axis sensor being each spaced approximately equidistant from each other around the generally circular shape of the expandable basket assembly.
Clause 34: The medical end effector according to any one of Clauses 19-33, wherein the first single-axis sensor, second single-axis sensor, and third single-axis sensor each comprise an inductive coil and wherein each inductive coil spirals around a respective coil axis such that each respective coil axis is approximately orthogonal to each spine of the plurality of spines that the first single-axis sensor, second single-axis sensor, and third single-axis sensor are each respectively coupled to.
Clause 35: The medical end effector according to Clause 34, wherein each respective coil axis extends towards the longitudinal axis and converge at a point generally disposed at that center of the expandable basket assembly.
Clause 36: The medical end effector according to any one of Clauses 19-35, wherein the first single-axis sensor, second single-axis sensor, and third single-axis sensor are disposed generally at the equator of the expandable basket assembly when in the expanded form.
Clause 37: The medical end effector according to any one of Clauses 19-36, further comprising a plurality of electrodes, each electrode of the plurality of electrodes being coupled to a spine of the plurality of spines.
Clause 38: The medical end effector according to Clause 37, wherein the first single-axis sensor, second single-axis sensor, and third single-axis sensor are coupled to a spine of the plurality of spines such that an electrode of the plurality of electrodes at least partially covers the first single-axis sensor.
Clause 39: The medical end effector according to Clause 19-38, wherein the plurality of spines consists of six spines.
Clause 40: The medical end effector according to Clause 39, wherein the first single-axis sensor, second single-axis sensor, and third single-axis sensor are coupled to every other spine of the six spines.
Clause 41: The medical end effector according to any of Clauses 19-40, wherein the plurality of spines is configured to form an approximately spherically-shaped basket assembly when in the expanded form.
Clause 42: The medical end effector according to any of Clauses 19-40, wherein the plurality of spines is configured form an approximately oblate-spheroid basket assembly when in the expanded form.

The embodiments described above are cited by way of example, and the present invention is not limited by what has been particularly shown and described hereinabove. Rather, the scope of the invention includes both combinations and sub combinations of the various features described hereinabove, as well as variations and modifications thereof which would occur to persons skilled in the art upon reading the foregoing description and which are not disclosed in the prior art.

## Claims

1. A medical end effector, comprising:
one or more spines extending along a longitudinal axis, the spines comprising:
a first surface being outward from the longitudinal axis;
one or more contact pads affixed to a portion of the first surface of the spine; and
one or more flexible circuits disposed over respective ones of the one or more contact pads, the one or more flexible circuits each comprising an electrode and an inductive coil as one unit.

2. The medical end effector according to Claim 1, the inductive coil comprising a first inductive coil embedded in the flexible circuit such that the first inductive coil conforms to a curvature of the flexible circuit bulging over respective ones of the ones of the one or more contact pads, optionally the first inductive coil being confined between two substantially parallel, flexible surfaces.

3. The medical end effector according to Claim 1, wherein the contact pad comprises a long side along the first surface and a short side perpendicular to the long side and the inductive coil is disposed solely on at least one of the long side and the short side.

4. The medical end effector according to Claim 1, wherein the electrode is disposed on a first side of the flexible circuit and the inductive coil is disposed on a second side of the flexible circuit, optionally wherein at least a portion of the first side of the flexible circuit is disposed generally parallel to the first surface of the spine or wherein at least a portion of the second side of the flexible circuit is disposed generally perpendicular to the first surface of the spine.

5. The medical end effector according to Claim 1, wherein each respective one of the one or more flexible circuits extends around the respective spine.

6. The medical end effector according to Claim 1, wherein the spines further comprise:
a second surface opposite the first surface;
a third surface disposed generally perpendicular to the first surface;
a fourth surface opposite the third surface,
wherein the one or more flexible circuits each extend around the first surface, third surface, fourth surface, and at least a portion of the second surface of the spine..

7. The medical end effector according to Claim 6, wherein the one or more flexible circuits extending around at least a portion of the second surface of the spine defines a gap in the flexible circuit through which one or more wires are routed.

8. The medical end effector according to Claim 1, further comprising:
an ablation power generator configured to be connected to the medical probe, and apply an electrical signal to at least one of the electrodes to ablate a tissue of a body part; and
a position module configured to receive position signals from at least one of the inductive coils.

9. The medical end effector according to Claim 1, wherein the one or more spines are configured to form an expandable basket assembly comprising the one or more spines extending along the longitudinal axis and configured to bow radially outward from the longitudinal axis when the expandable basket assembly is transitioned from a collapsed form to an expanded form.

10. The medical end effector according to Claim 1 further comprising a three-axis sensor coupled to the one or more spines wherein the one or more spines comprises a distal end and a proximal end and the three-axis sensor is disposed proximate the distal end.

11. A medical end effector comprising:
an expandable basket assembly comprising:
a plurality of spines extending along a longitudinal axis and configured to bow radially outward from the longitudinal axis when the expandable basket assembly is transitioned from a collapsed form to an expanded form;
a first single-axis sensor;
a second single-axis sensor; and
a third single-axis sensor,
the first single-axis sensor, second single-axis sensor, and third single-axis sensor are each coupled to a respective spine of the plurality of spines and are collectively configured to function as a three-axis sensor; and
a processor configured to:
receive position data from the first single-axis sensor, second single-axis sensor, and third single-axis sensor;
receive known information pertaining to physical properties of the spines of the expandable basket assembly; and
calculate a position of the expandable basket assembly and a force applied to the expandable basket assembly based at least in part on the received position data and known information pertaining to the physical properties of the spines.

12. The medical end effector according to Claim 11, wherein the processor is further configured to:
calculate a deflection of the expandable basket assembly at least in part on received position data,
optionally wherein the processor is further configured to:
calculate a force applied to the expandable basket assembly based at least in part on the calculated deflection of the expandable basket assembly and the known information pertaining to the physical properties of the spines.

13. The medical end effector according to Claim 11, wherein the processor is further configured to:
calculate a deflection of one or more individual spines of the plurality of spines at least in part on received position data.

14. The medical end effector according to Claim 13, wherein the processor is further configured to:
calculate an individual force applied to each of one or more individual spines of the plurality of spines based at least in part on the calculated deflection of one or more individual spines and the known information pertaining to the physical properties of the spines.

15. The medical end effector according to Claim 11, further comprising a plurality of strain gauges, each strain gauge of the plurality of strain gauges coupled to a spine of the plurality of spines., optionally wherein the processor is further configured to:
receive force data from the plurality of strain gauges; and
calculate a force and a direction of said force applied to the expandable basket assembly based at least in part on the received force data from the plurality of strain gauges.
